# EUROPEAN PATENT APPLICATION

(11) **EP 2 589 963 A1**
(43) Date of publication of application: **08.05.2013**
(21) Application number: 11306438.0
(22) Date of filing: 04.11.2011
(51) Int. Cl.: G01N 33/574, A61K 39/395, A61P 35/00

(54) **Diagnostic and treatment of an androgen-independent prostate cancer**

(71) Applicant: Université de Provence (Aix-Marseille 1), 13331 Marseille Cedex 3 (FR)
(72) Inventor: Berenguer-Daize, Caroline, 13011 Marseille (FR); Martin, Pierre-Marie, 13008 Marseille (FR); Mabrouk, Kamel, 13170 Les Pennes Mirabeau (FR); Bertin, Denis, 13013 Marseille (FR); Ouafik, L'Houcine, 13005 Marseille (FR)
(74) Representative: Rançon, Xavier Lucien Abel

(57) **Abstract**

The present invention relates to a method for monitoring the tumor growth of an androgen-independent prostate cancer in a subject, by determining the amount of adrenomedullin and/or at least one adrenomedullin receptor in a prostate cancer cell obtained from said subject. The present invention also relates to a pharmaceutical composition comprising an anti-adrenomedullin antagonist antibody and/or at least one anti-adrenomedullin receptor antagonist antibody for treating an androgen-independent prostate cancer.

SEQUENCE LISTING

<110>  UNIVERSITE DE PROVENCE - AIX-MARSEILLE 1
       BERENGUER-DAIZE, Caroline
       MARTIN, Pierre-Marie
       MABROUK, Kamel
       BERTIN, Denis
       OUAFIK, L'Houcine

<120>  DIAGNOSTIC AND TREATMENT OF AN ANDROGEN-INDEPENDENT PROSTATE
       CANCER

<130>  XRN-clg-F2245-3

<160>  9

<170>  PatentIn version 3.5

<210>  1
<211>  24
<212>  PRT
<213>  Artificial Sequence

<220>
<223>  peptide derived from adrenomedullin

<400>  1

Arg Ser Phe Arg Phe Gly Lys Leu Ala Thr Asp Lys Asp Lys Asp Asn
1               5                   10                  15

Val Ala Pro Arg Ser Lys Ile Ser
            20

<210>  2
<211>  25
<212>  PRT
<213>  Artificial Sequence

<220>
<223>  peptide derived from hCLR

<400>  2

Ser Pro Glu Asp Ser Ile Gln Leu Gly Val Thr Arg Asn Lys Ile Met
1               5                   10                  15

Thr Ala Gln Tyr Glu Ala Tyr Gln Lys
            20                  25

<210>  3
<211>  31
<212>  PRT
<213>  Artificial Sequence

<220>
<223>  peptide derived from hCLR

<400>  3

Pro Asp Tyr Phe Gln Asp Phe Asp Pro Ser Glu Lys Val Thr Lys Ile

1               5                   10                  15

Ala Asp Gln Asp Gly Asn Trp Phe Arg His Pro Ala Ser Asn Arg
            20                  25                  30

<210>  4
<211>  23
<212>  PRT
<213>  Artificial Sequence

<220>
<223>  peptide derived from hRAMP2

<400>  4

Lys Asn Tyr Glu Thr Ala Val Gln Phe Ala Trp Asn His Tyr Lys Asp
1               5                   10                  15

Gln Met Asp Pro Ile Glu Lys
            20

<210>  5
<211>  28
<212>  PRT
<213>  Artificial Sequence

<220>
<223>  peptide derived from hRAMP2

<400>  5

Arg Pro Tyr Ser Thr Leu Arg Asp Ala Leu Glu His Phe Ala Glu Leu
1               5                   10                  15

Phe Asp Leu Gly Phe Pro Asn Pro Leu Ala Glu Arg
            20                  25

<210>  6
<211>  6
<212>  PRT
<213>  Artificial Sequence

<220>
<223>  peptide derived from hRAMP3

<400>  6

Lys Val Asp Val Trp Lys
1               5

<210>  7
<211>  22
<212>  PRT
<213>  Artificial Sequence

<220>
<223>  peptide derived from hRAMP3

<400>  7

Leu Glu Arg Leu Pro Leu Ala Gly Lys Ala Phe Ala Asp Met Met Gly
1               5                   10                  15

Lys Val Asp Val Trp Lys
            20

<210>  8
<211>  22
<212>  PRT
<213>  Artificial Sequence

<220>
<223>  peptide derived from hRAMP3

<400>  8

Gly Phe Ile Thr Gly Ile His Arg Gln Phe Phe Ser Asn Ala Thr Val
1               5                   10                  15

Asp Arg Val His Leu Glu
            20

<210>  9
<211>  78
<212>  PRT
<213>  Artificial Sequence

<220>
<223>  chimeric peptide

<400>  9

Arg Ser Phe Arg Phe Gly Lys Leu Ala Thr Asp Lys Asp Lys Asp Asn
1               5                   10                  15

Val Ala Pro Arg Ser Lys Ile Ser Pro Asp Tyr Phe Gln Asp Phe Asp
            20                  25                  30

Pro Ser Glu Lys Val Thr Lys Ile Ala Asp Gln Asp Gly Asn Trp Phe
        35                  40                  45

Arg Lys Val Asp Val Trp Lys Lys Asn Tyr Glu Thr Ala Val Gln Phe
    50                  55                  60

Ala Trp Asn His Tyr Lys Asp Gln Met Asp Pro Ile Glu Lys
65                  70                  75

## Description

The present invention relates to markers for diagnosing an androgen-independent prostate cancer tumor growth, and the use of antibodies anti-adrenomedullin and/or anti-adrenomedullin receptor for treating an androgen independent prostate cancer.

Prostate cancer (CaP) is the most-diagnosed malignant growth in men and is the second-leading cause of male cancer deaths in the majority of Western countries. The cancerous gland usually contains multiple independent and genetically distinct lesions, demonstrating heterogeneity of the disease (1, 2). Because pathologic growth of the prostate is controlled largely by steroid androgens, treatment of locally advanced or metastatic disease relies heavily on hormonal therapies that target the androgen receptor (AR). A major limitation of hormonal therapy, however, is that it offers only temporary relief; the cancer eventually reappers as an androgen-independent (AI) lesion characterized by aggressive growth and invasion of distal organs (3). Whether it is clonal expansion or adaptation, development of AI prostate cancer clearly shows that those factors other than, or together with, androgen must exist to provide survival and growth instructions to the AI cells.

Therefore, there is a need for accurate diagnosis markers of AI prostate cancer.

Rocchi *et al.* have reported the expression and characterization of adrenomedullin (AM) in the prostate-cancer patients with high Gleason's score suggesting a role of AM in prostate cancer growth and hormonal escape (4). At the time of its discovery AM was noted to be a potent vasodilator (5), and it has subsequently been linked to the regulation of numerous vascular responses, including blood pressure, salt and water intake, and angiogenesis (6-8), as well as regulator of cell growth (7, 9-11). These functions were discovered due to many physiological studies and are highlighted in knockout studies in which AM null mice die *in utero* from extreme hydrops fetalis, vascular and lymphatic abnormalities (12, 13). AM is expressed in a variety of malignant tissues and was shown to be mitogenic for human cancer cell lines including lung, breast, colon, glioblastoma, pancreas, kidney and prostate lineages *in vitro* (4, 8, 14-19). Evidence for the importance of AM-induced angiogenesis in tumor growth is that inhibition of AM action by neutralizing antibodies or AM antagonist (hAM₂₂₋₅₂) blocks the growth of tumor xenografts *in vivo* (15, 16). The density of blood vessels in anti-AM antibody-treated tumors is decreased; supporting the notion that AM plays roles in angiogenesis, vasculogenesis and/or vessel stabilization (7, 8, 11, 15, 20, 21). The coupling of these functions with the potent effects of AM on cell migration, growth, and apoptosis have led to the hypothesis that AM may be a key player in tumor growth and metastasis (22, 23).

AM transduces its effects through the G protein-coupled receptor calcitonin receptor-like receptor (CLR), with specifity for AM being conferred by the receptor activity modifying protein -2 (RAMP2) and -3 (RAMP3) (24). The ability of CLR/RAMP2 and CLR/RAMP3 to respond with high affinity to AM implies the existence of two molecularly distinct AM receptors referred as AM₁ and AM₂ receptors, respectively (25). Mice deficient in the components of AM receptors, CLR and RAMP2 develop edema and embryonic lethality at midgestation (26). Another study of RAMP2 gene-targeted mice concludes that the embryonic edema observed in these mice is due to hyperpermeable blood vessels (27). Multiple second messenger responses to AM mediate a multitude of biological functions (6, 10). The activation of the receptor by AM has commonly been linked to elevation of cAMP (10). There are reports demonstrating that AM can signal through other mechanisms such as elevation of free intracellular Ca²⁺ levels (28), activation of cGMP-NO pathway (10), activation of cGMP-PKG pathway (29), Ras-Raf pathway (21), K⁺-ATP channels (30), or PI3-kinase/Akt, FAKinase and MAPkinase pathways (20).

It has previously been reported the presence of AM in human prostate tissues, prostate carcinoma, and prostate cancer cell lines (4, 31, 32). Some of the inventors have shown an increase in AM levels in LuCaP 23.1 xenografted tumors after castration (33). Abasalo *et al.* have shown that AM prevents apoptosis in prostate cancer cells (34). Recently, Joshi *et al.* demonstrated that AM up-regulates interleukin-13 receptor α2 chain in prostate cancer *in vitro* and *in vivo* revealing a novel role of AM in sensitizing certain types of prostate tumors to IL-13R-directed therapeutic agent (35). Some of the inventors have reported that the expression of AM is down-regulated by androgen in androgen-sensitive LNCaP cells *in vitro* and *in vivo.* Further, in the absence of androgen, AM promotes prostate cancer tumor growth (29).

The inventors have studied the cellular and molecular mechanisms activated by AM to promote CaP growth in androgen-independent manner. The inventors have used the hormone independent prostate cancer cell line Du145 as model to investigate the expression of AM system (AM, AM₁ and AM₂ receptors) and to evaluate its role *in vitro* as well as *in vivo.* The effect of anti-AM polyclonal antibody (αAM) on tumor growth was further studied in a mouse heterotopic and orthotopic prostate-cancer xenograft models.

The inventors have shown that αAM decreases the proliferation and invasion of Du145 cells *in vitro* and inhibits the growth of androgen-independent Du145 tumor xenografts by targeting tumor angiogenesis and lymphangiogenesis. The inventors have also shown that AM is one of the major factor(s) involved in the lymphatic vessels generation and/or lymphatic vessel stabilization.

These results support that interference with the function of AM following androgen ablation represent a tractable system for the treatment of CaP growth at the hormone-independent escape and inhibition of tumor metastasis.

These results support further that determining the amount of adrenomedullin and/or at least one adrenomedullin receptor in a prostate cancer cell can be useful for diagnosing a regression or a progression of an androgen-independent prostate cancer tumor growth. A subject, for which an androgen-independent prostate cancer tumor growth progression has been diagnosed cab be treated with an anti-adrenomedullin antagonist antibody and/or at least one anti-adrenomedullin receptor antagonist antibody.

Accordingly, in a first aspect, the present invention provides a method of monitoring the tumor growth of an androgen-independent prostate cancer in a subject, comprising the step of:
a) determining the amount of adrenomedullin and/or at least one adrenomedullin receptor selected among the proteins CLR, RAMP2 and RAMP3, in a prostate cancer cell obtained from said subject, and
b) comparing the amount determined in step a) with amounts previously obtained for the subject or with a standard,
a decrease in amount of adrenomedullin and/or at least one adrenomedullin receptor constituting a marker of the regression of said androgen-independent prostate cancer tumor growth and an increase in amount of adrenomedullin and/or at least one adrenomedullin receptor constituting a marker of the progression of said androgen-independent prostate cancer tumor growth.

The prostate cancer cells are generally obtained from a prostate tissue sample carried out by biopsy.

As used herein, the term "subject" refers to mammal, preferably a human.

As used herein, the term "adrenomedullin receptor" refers to the proteins CLR, RAMP2 and RAMP3 respectively. These proteins can be in a form of a protein complex CLR/RAMP2, CLR/RAMP3, RAMP2/RAMP3 and CLR/RAMP2/RAMP3. These proteins CLR, RAMP2, RAMP3 are well known to a person skilled in the art (24). By way of example, an amino acid sequence of the proteins CLR, RAMP2, RAMP3 are available under the accession numbers gi|5031621, gi|118572585 and gi|5032023 in the GENBANK database respectively.

Advantageously, the amount of adrenomedullin and of at least one, preferably at least two, more preferably the three adrenomedullin receptor(s) is determined.

As used herein, the term "standard" refers to the amount of adrenomedullin and/or at least one adrenomedullin receptor in said prostate cells which has been determined in a large population of subjects not suffering from an androgen-independent prostate cancer.

The method according to the invention can be performed *in vitro*, *ex vivo* or *in vivo.*

In a preferred embodiment of step a) of the method according to the present invention, said tumor cell is contacted with an antibody or antibodies binding adrenomedullin and/or at least one adrenomedullin receptor.

The antigen-antibody complexes formed in step a) can then be revealed, by any suitable means, for example by EIA, ELISA, RIA, immunofluorescence or immunohistochemistry, preferably immunohistochemistry.

As used herein, the term "antibody" encompasses polyclonal or monoclonal antibodies, natural, synthetic or recombinant antibodies, camelid single-domain antibodies, chimeric antibodies such as a humanized antibodies, and the fragments thereof (*e.g.* Fab'2, Fab, Fv, scFv) having retained their ability to bind adrenomedullin, CLR, RAMP2 and/or RAMP3.

The term "recombinant antibody" is intended to mean an antibody produced by genetic engineering, for example by cloning and gene amplification

The term "synthetic antibody" is intended to mean an antibody produced by enzymatic and/or chemical synthesis.

The term "chimeric antibody" is intended to mean an antibody from a particular animal species or a particular class of antibodies, which includes all or part of a heavy chain and/or light chain of an antibody from another species or another antibody class. The term "chimeric antibody" may also designate a multispecific antibody, *i.e.*, specific to at least two different epitopes. The multispecific antibodies can be obtained from said antibody fragments as defined above. Advantageously, said multispecific antibody binds to two or three different epitopes selected from an epitope from adrenomedullin, an epitope from CLR, an epitope from RAMP2 and an epitope from RAMP3. Therefore, an anti-adrenomedullin antagonist antibody and an anti-adrenomedullin receptor antagonist antibody can be in a form of single multispecific antibody. By way of example a bi-specific antibody includes an antibody which binds to a complex selected from AM/CLR, AM/RAMP2, AM/RAMP3, CLR/RAMP2, CLR/RAMP3 and RAMP2/RAMP3, preferably a complex selected among CLR/RAMP2, CLR/RAMP3 and RAMP2/RAMP3. By way of example, a tri-specific antibody includes an antibody which binds to a complex selected among AM/CLR/RAMP2, AM/CLR/RAMP3, AM/RAMP2/RAMP3 and CLR/RAMP2/RAMP3, preferably CLR/RAMP2/RAMP3 complex. Advantageously again, said multispecific antibody binds to adrenomedullin and the proteins CLR, RAMP2 and RAMP3.

The term "humanized antibody" refers to a human immunoglobulin in which the residues of the CDRs (Complementary-Determining Region) that form the binding site to the antigen are replaced by those of a non-human monoclonal antibody with a desired specificity, affinity and/or activity.

The antibodies and antibody fragments can be prepared by conventional techniques well known to a person skilled in the art. By way of example, anti-AM, -CLR, -RAMP2 or -RAMP3 antibodies can be obtained as described by Fernandez-Sauze *et al.* (7), Ouafik *et al.* (15) and in International Applications WO 2007/045927 and WO 2010/012911.

Advantageously, said antibodies can be obtained by immunizing a suitable animal with a chimeric polypeptide (antigen) comprising or consisting of four peptides of at least 6 contiguous amino acid, derived from adrenomedullin and an extracellular domain of the proteins CLR, RAMP2 and RAMP3 respectively, said chimeric polypeptide being capable of inducing, in said animal immunized with said chimeric polypeptide, the production of polyclonal antibodies that bind to adrenomedullin, CLR, RAMP2 and RAMP3. The immunized animal can be a mammal, such as a horse, goat, rabbit, rat, mouse, llama and camel.

A chimeric polypeptide (antigen) as defined above, which mimics the structure of the extracellular domains of proteins CLR, RAMP2 and RAMP3, can induce the production in an immunized animal, of a conformational antagonist antibody that specifically recognize these proteins CLR, RAMP2 and RAMP3 in their native form or protein complexes, that is to say, the complex AM/CLR, AM/RAMP2, AM/RAMP3, CLR/RAMP2, CLR/RAMP3, RAMP2/RAMP3, AM/CLR/RAMP2, AM/CLR/RAMP3, AM/RAMP2/RAMP3, CLR/RAMP2/RAMP3 or AM/CLR/RAMP2/RAMP3.

Advantageously, each of the following peptides can be used to immunize an animal or to produce a chimeric polypeptide useful for immunizing an animal, for obtaining antibodies as defined above:
- peptide derived from adrenomedullin, consisting of the concatenation of fragments F14-R12, R17, G19, K25-A27 and T34-S48 of adrenomedullin:
   RSFRFGKLATDKDKDNVAPRSKIS (SEQ ID NO: 1, referred to as AM24)
- peptides derived from fragments S27-K51 (SEQ ID NO: 2) or P89-R119 (SEQ ID NO: 3) of the protein hCLR:
   SPEDSIQLGVTRNKIMTAQYEAYQK (SEQ ID NO: 2)
   PDYFQDFDPSEKVTKIADQDGNWFRHPASNR (SEQ ID NO: 3),
- peptides derived from fragments K59-K81 (SEQ ID NO: 4) or R91-R118 (SEQ ID NO: 5) of the protein hRAMP2:
   KNYETAVQFAWNHYKDQMDPIEK (SEQ ID NO: 4),
   RPYSTLRDALEHFAELFDLGFPNPLAER (SEQ ID NO: 5),
- peptides derived from fragments K49-K55 (SEQ ID NO: 6), A34-K55 (SEQ ID NO: 7) or G91-E112 (SEQ ID NO: 8) of the protein hRAMP3:
   KVDVWK (SEQ ID NO: 6),
   LERLPLAGKAFADMMGKVDVWK (SEQ ID NO: 7),
   GFITGIHRQFFSNATVDRVHLE (SEQ ID NO: 8).

The order of the different peptides derived from adrenomedullin and adrenomedullin receptors CLR, RAMP2 and RAMP3 respectively may be randomly chosen for producing the chimeric polypeptide. However, it is preferable to produce a chimeric polypeptide comprising or consisting, from the N-terminus to the C-terminus, a peptide derived from adrenomedullin, a peptide derived from CLR, a peptide derived from RAMP3 and a peptide derived from RAMP2. Preferably, the chimeric polypeptide (antigen) has the amino acid sequence SEQ ID NO: 9 (referred to as ch2):
RSFRFGKLATDKDKDNVAPRSKISPDYFQDFDPSEKVTKIADQDGNWFRKVD VWKKNYETAVQFAWNHYKDQMDPIEK.

Said antibody can be linked, directly or indirectly, covalently or non-covalently to a diagnostic agent selected from the group consisting of:
- enzymes such as horseradish peroxidase, alkaline phosphatase, glucose-6-phosphatase or beta-galactosidase;
- fluorophores such as green fluorescent protein (GFP), blue fluorescent dyes excited at wavelengths in the ultraviolet (UV) part of the spectrum (*e.g.* AMCA (7-amino-4-methylcoumarin-3-acetic acid); Alexa Fluor 350), green fluorescent dyes excited by blue light (e.g. FITC, Cy2, Alexa Fluor 488), red fluorescent dyes excited by green light (*e.g.* rhodamines, Texas Red, Cy3, Alexa Fluor dyes 546, 564 and 594), or dyes excited with far-red light (*e.g.* Cy5) to be visualized with electronic detectors (CCD cameras, photomultipliers);
- heavy metal chelates such as europium, lanthanum or yttrium; and
- radioisotopes such as [¹⁸F]fluorodeoxyglucose, ¹¹C-, ¹²⁵I-, ¹³¹I-, ³H-, ¹⁴C-, ³⁵S, or ⁹⁹Tc- labelled compounds.

Said diagnostic agent can be directly and covalently linked to said antibody either to one of the terminal ends (N or C terminus) of the antibody, or to the side chain of one of the amino acids of the antibody. The substance of interest can also be indirectly and covalently linked to said antibody by a connecting arm *(i.e.,* a cross-linking reagent) either to one of the terminal ends of the antibody or to a side chain of one of the amino acids of the antibody. Linking methods of a substance of interest to an antibody are known in the art.

Many chemical cross-linking methods are also known in the art. Cross-linking reagents may be homobifunctional (*i.e.*, having two functional groups that undergo the same reaction) or heterobifunctional (*i.e.*, having two different functional groups). Numerous cross-linking reagents are commercially available. Detailed instructions for their use are readily available from the commercial suppliers.

Methods for detecting and quantifying adrenomedullin and adrenomedullin receptors by using anti-adrenomedullin and anti-adrenomedullin receptor antibodies are known to a person skilled in the art (7, 15).

In another preferred embodiment of step a) the method according to the present invention, when it is carried *in vitro* or *ex vivo,* the amount of adrenomedullin and/or of at least one adrenomedullin receptor in a prostate cancer cell is determined by detecting and quantifying RNA sequences coding adrenomedullin and/or an adrenomedullin receptor, by using a quantitative reverse transcriptase polymerase chain reaction (Q-RT-PCR) assay.

Methods for detecting and quantifying RNA sequences coding adrenomedullin or an adrenomedullin receptor by using a Q-RT-PCR assay are known to a person skilled in the art (4, 15, 29).

In a second aspect, the present invention provides a pharmaceutical composition comprising an anti-adrenomedullin (anti-AM) antagonist antibody and/or at least one anti-adrenomedullin receptor (anti-AMR) antagonist antibody for use as a medicament for treating an androgen-independent prostate cancer.

The anti-AM and anti-AMR antibodies of said composition are antagonist antibodies possessing the following properties:
- they bind specifically to AM or an AMR and possess a high affinity for AM or AMR, and
- they are capable of inhibiting the binding between AM and an AMR in a dose-dependent manner.

These properties can be verified by conventional tests which make it possible to evaluate the binding activity of an antibody to a protein or to a peptide or the inhibition of this activity, in particular by direct and competition ELISA tests.

Advantageously, said pharmaceutical composition also comprises a pharmaceutically acceptable carrier.

As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field. Preferred examples of such carriers or diluents include, but are not limited to, water, saline, Ringer's solutions, dextrose solution, and 5% human serum albumin. Liposomes, cationic lipids and non-aqueous vehicles such as fixed oils may also be used. The use of such media and agents for pharmaceutically active substances is well known in the art. Except insofar as any conventional media or agent is incompatible with a therapeutic agent as defined hereabove, use thereof in the composition of the present invention is contemplated.

In addition to the preceding features, the invention further comprises other features which will emerge from the following description, which refers to examples illustrating the present invention, as well as to the appended figures.

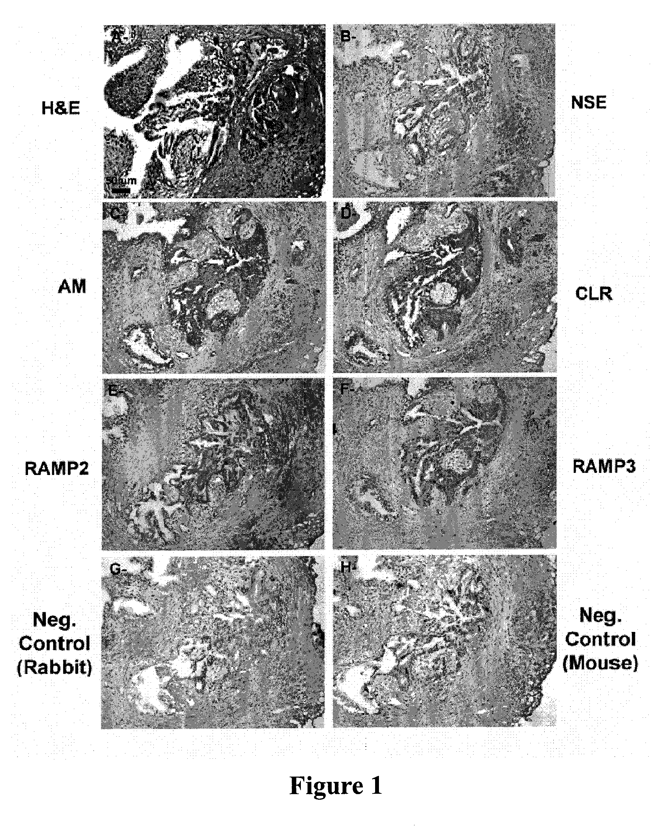

**Figure 1****: AM, CLR, RAMP2 and RAMP3 are expressed in prostate cancer tissues.** The great majority of both epithelial and stromal cells showed NSE (neuron-specific enolase) staining (**B**) suggesting a neuroendocrine differentiation of cancer specimen. Immunohistochemistry showing the expression of AM (**C**)**,** CLR (**D**)**,** RAMP2 (**E**) and RAMP3 (**F**) proteins in the cancer epithelium of prostate. Orthotopic tumor sections stained with hematoxylin and eosine (H&E) (**A**)**.** CLR staining of cells dispersed among the stroma can be observed (**D**)**.**

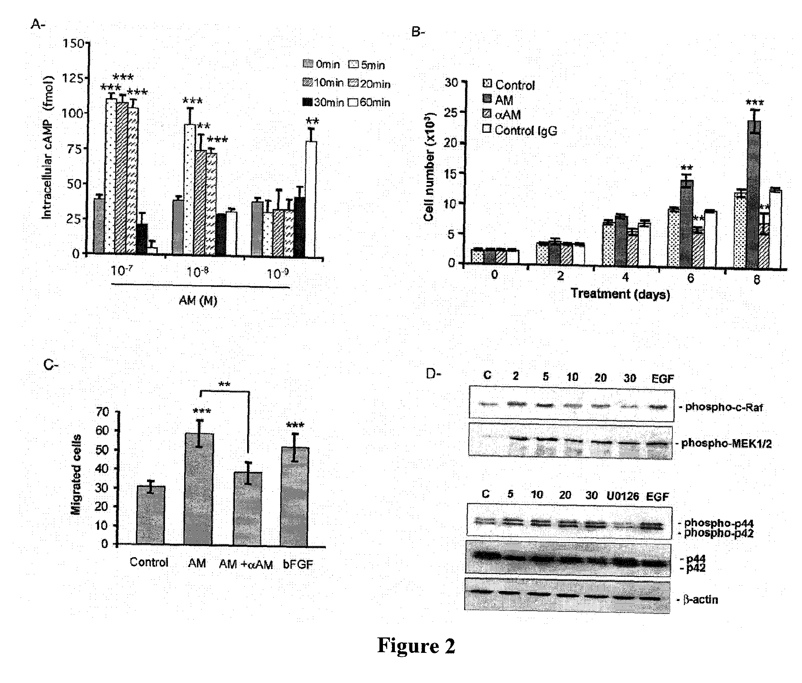

**Figure 2****: Adrenomedullin stimulates Du145 cell proliferation and invasion *in vitro.* A,** AM induced cAMP formation in cultured Du145 cells. Cells (3 x 10⁴ cells/ml) were treated with AM (10⁻⁹ M-10⁻⁷ M) for different times. Each value is the mean ± SEM of three independent experiments performed in quadruplate *(*, p* < 0.05; **, *p* < 0.01; ****, p* < 0.001). **B,** oppose effects of AM and αAM on the growth of Du145 cells *in vitro.* For proliferation assays, Du145 cells were seeded at the density of 1 x 10⁴ per well in 6 multiwell plates in presence of RPMI 1640 medium supplemented with 5% FBS. AM at 10⁻⁷ M and αAM (70µg/ml) were added. As control, rabbit control IgG (70µg/ml) of irrelevant specificity was used. For each treatment, three wells were prepared, exposed to trypsin and cells were counted in Coulter Counter. Each *bar* represents the mean ± SEM of four independent experiments *(**, p* < 0.01; ****, p* < 0.001). **C,** AM stimulates Du145 cells invasion *in vitro.* Du145 cells (1.5 x 10⁴ cells) were added into Biocoat Matrigel invasion upper chamber and 10⁻⁷ M of AM were added into the lower chamber, and after 6 h, noninvaded cells in the upper membrane were removed and cells invading into the lower membrane were quantified by fluorescence by adding DAPI reagent at least 45 min before taking the reading. RPMI 1640 containing 2% FBS was used as control. bFGF was used as positive control. Data are expressed as the number of migrated cells in 10 high-power fields and are the means of results from three independent experiments. Each *bar* represents the mean ± SEM (**, *p* < 0.01; ****, p* < 0.001). **D,** intracellular signalling pathway induced by AM in Du145 cells. Western blot analysis of phosphorylated cRAF (pcRAF), pMEK1/2, pERK (44 and 42 kDa) and ERK in Du145 cells, stimulated with AM (5 x 10⁻⁷ M) for 2, 5, 10, 20, and 30 min. AM (5 x 10⁻⁷ M)-induced phosphorylation of ERK was inhibited by mitogen-activated protein kinase kinase inhibitor, U0126 (10 µM, 30 min). EGF was used as positive control known to stimulate phosphorylation of cRAF, MEK, and ERK. To assess the amount of proteins loaded for each sample, immunoblot analysis was performed with anti-α-actin.

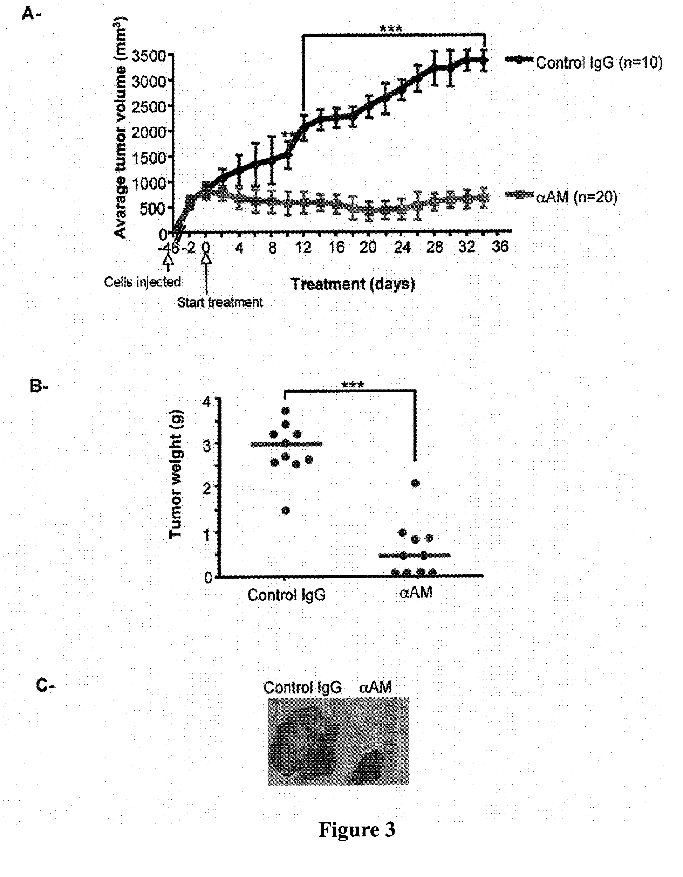

**Figure 3****: Growth of heterotopic and orthotopic Du145 tumor xenografts in nude mice are inhibited by αAM. A**, human prostate tumors were established by injecting athymic nude mice s.c. in the right flank with 5 x 10⁶ Du145 cancer cells. Tumors were allowed to reach ∼700 mm³ in size, and then randomized groups of mice received i.p. injections of either αAM or control IgG (330 µg/dose) every 3 days. Tumor volumes were measured twice weekly, as described in Materials and Methods. Values are means ± SEM. *Asterisks* indicate that the value is significantly different from the control-IgG (**, *p* < 0.01; ***, *p* < 0.001). **B,** orthotopic human prostate tumors were established by injection of 1 x 10⁶ Du145 cancer cells in the dorsal lobe of prostate. After 30 days, mice were randomized and treated as above. Tumor weight of αAM-treated and control IgG animals is shown and the mean tumor weight in each treatment group is indicated by horizontal lines. **C,** representative images of orthotopic tumor xenografts from αAM-treated and control IgG animals.

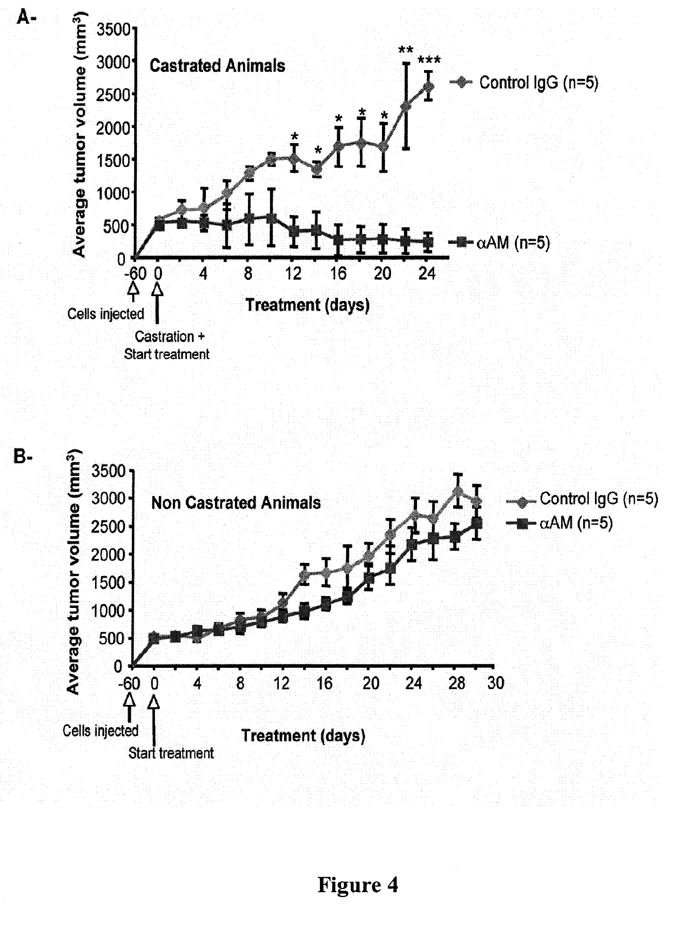

**Figure 4****: The effect of αAM on tumor growth of LNCaP xenografts in castrated and non-castrated animals.** The LNCaP cells (5x10⁶) in Matrigel (v/v) were subcutaneously implanted in *nu*/*nu* mice. After 3 months, when the primary tumors were 400-500 mm³ in size, a group of animals (n=10) was castrated and randomly divided into two groups that received αAM or IgG-control by i.p. administration. A second group of animals (n=10) was non castrated and randomly divided into two groups and treated as above. The sizes of the LNCaP xenografts were determined as described in Fig. 3. The asterisks indicate that the tumor volume αAM treated xenografts is significantly different from control IgG treated xenografts in castrated animals (**, p* < 0.05;**, *p* < 0.01;***, *p* < 0.001).

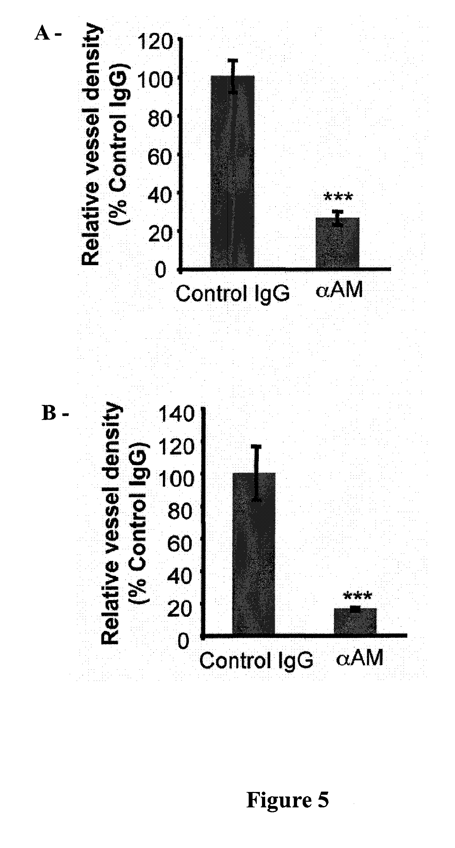

**Figure 5****: Depletion of endothelial cells in αAM-treated tumors.** To visualize functional blood vessels in tumors, tumor sections (6 µm) from subcutaneous or orthotopic xenografts were evaluated by immunofluorescence for vWF, a marker to detect endothelial cells, and αSMA, a marker for mature pericytes. Tissue sections were counterstained with DAPI. Quantitative assessment of cell density of cells that stained positive for vWF in heterotopic (**A**) and orthotopic (**B**) xenografts was conducted through a microscope. Image J 1.43U software was used for analysis. Values ± SEM; n=6. ***, *p* < 0.001.

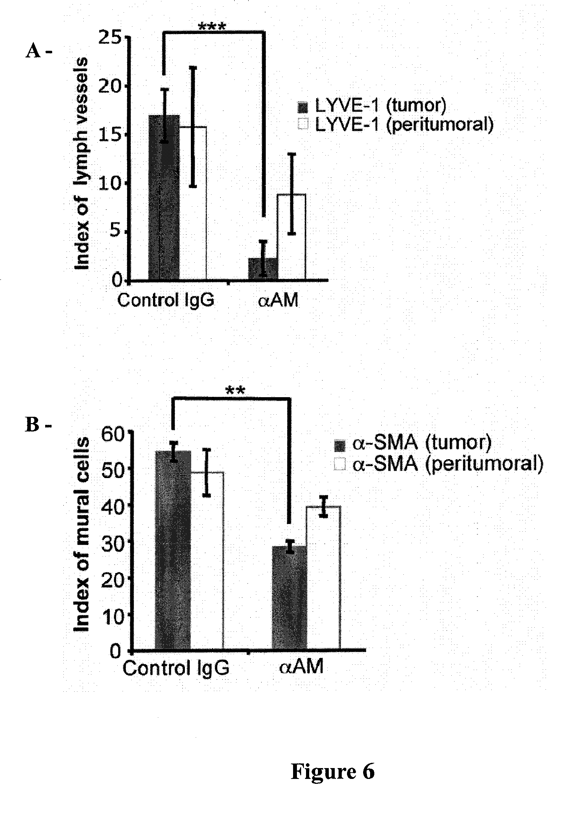

**Figure 6****: αAM inhibits tumor-associated lymphangiogenesis in Du145 prostate tumor model.** After 40 days treatment, Du145 primary tumors from IgG-control and αAM-treated mice were harvested and analyzed for lymphatic vessels using a murine LYVE-1 antibody (DAB) and for mural lymphatic cells using anti-αSMA antibody by immunohistochemistry. **A,** immunostained vessel structures was enumerated from eight sections of four representative Du145 tumors in each experimental group. Values are means ± SEM; ***; *p* < 0.001. **B,** quantitative assessment of mural cell density that stained positive for αSMA was conducted through a microscope and analyzed using Image J 1.43U software. Values are means ± SEM; ***; *p* < 0.001.

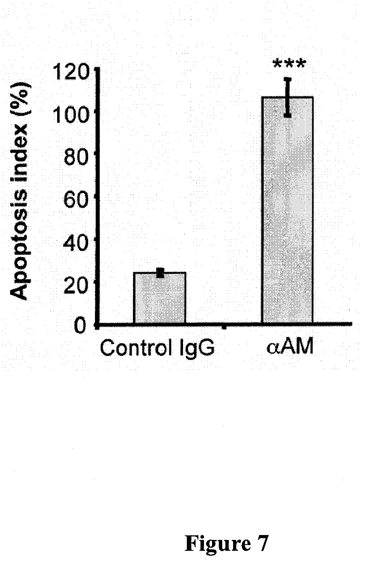

**Figure 7****: αAM treatment induced LECs-associated tumors apoptosis.** Du145 cells (2x10⁶) were implanted into prostate of athymic (nu/nu) mice. After treatment for 40 days with αAM or IgG-control, animals were sacrificed, and orthotopic Du145 prostate were harvested. To visualize the nature of cells undergoing apoptosis in tumor, tumor sections (6 µm) were evaluated by immunofluorescence for LYVE-1, a marker to detect lymphatic endothelial cells, and ssDNA, a marker for cells undergoing apoptosis Quantitative assessment of cell density of cells that stained positive for ssDNA was conducted through a microscope. Image J 1.43U software was used for analysis. Values are means ± SEM; ***; *p* < 0.001.

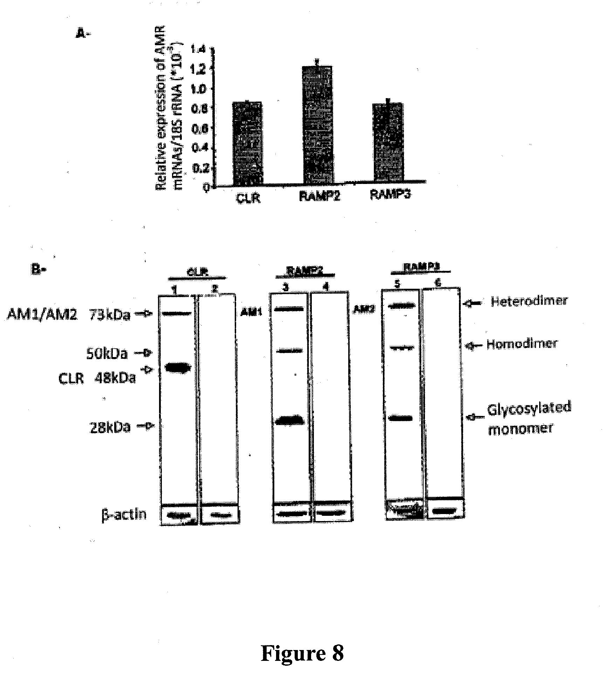

**Figure 8****: Adrenomedullin receptors expression in prostate cancer cells Du145. A,** total RNA (1 µg) DNA-free from Du145 cell line was reverse transcribed into cDNA and subjected to real-time quantitative reverse transcription (RT)-PCR for the estimation of relative CLR, RAMP2 and RAMP3 mRNAs to 18S rRNA ratio. **B**, aliquots of membrane preparations (50 µg) of Du145 cells (lanes 1, 3, and 5) were separated on 12% SDS-PAGE, transferred to Hybond-C membrane, and immunoblotted using antibodies generated against CLR, RAMP2, and RAMP3 peptides. Staining for all samples was blocked by addition of 20 µg/ml CLR, RAMP2 and RAMP3 peptides (lanes 2, 4, and 6). To assess the amount of proteins loaded for each sample, immunoblot analysis was performed with anti-α-actin. Du145 cells produced a CLR as a distinct band of 48 kDa and multimer, presumably heterodimers CLR/RAMP2 (AM₁ receptor) or CLR/RAMP3 (AM₂ receptor), at 73-76 kDa (Figure 8B, lane 1). RAMP2 and RAMP3 were seen as a monomer of 28 kDa and multimer, presumably homodimer at 50 kDa and heterodimer at 73-76 kDa (AM₁ and AM₂ receptors revealed with anti-RAMP2 and anti-RAMP3 antibodies, respectively) (Figure 8B, lanes 3 and 5). Together, these findings demonstrated that CLR, RAMP2 and RAMP3 mRNAs and proteins are expressed in androgen-independent Du145 cells.

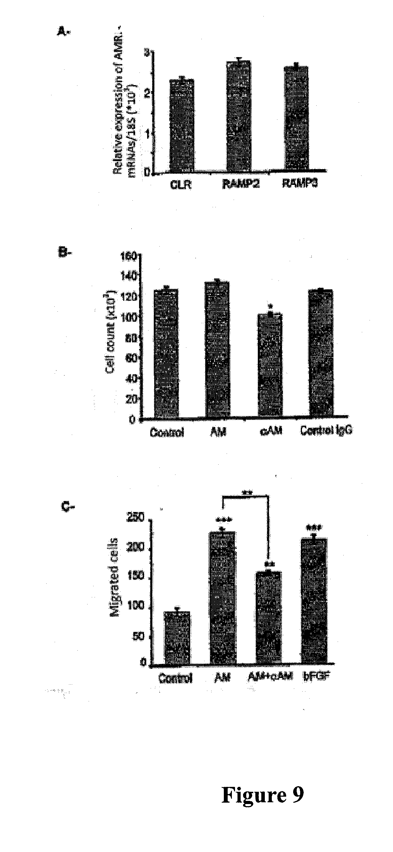

**Figure 9****: Effect of AM on PC3 cells *in vitro.* A,** expression of AMR mRNAs in PC3 cells. Total RNA (1 µg) DNA-free from PC3 cell line was reverse transcribed into cDNA and subjected to real-time quantitative reverse transcription (RT)-PCR for the estimation of relative CLR, RAMP2 and RAMP3 mRNAs to 18S rRNA ratio. **B**, effects of AM and αAM on the growth of PC3 cells *in vitro.* For proliferation assays, PC3 cells were seeded at the density of 2 x 10⁴ per well in 24 multiwell plates in the presence of RPMI 1640 medium supplemented with 2% FBS. AM at 10⁻⁷M or αAM (70µg/ml) were added. As control, rabbit control IgG (70µg/ml) of irrelevant specificity was used. For each treatment, six wells were prepared, exposed to trypsin and cells were counted in Coulter Counter. Each *bar* represents the mean ± SEM of four independent experiments. **C**, AM stimulates PC3 cells invasion *in vitro.* PC3 cells (3 x 10⁴ cells) were added into Biocoat Matrigel invasion upper chamber and 10⁻⁷ M of AM were added into the lower chamber, and after 6 h, non-invaded cells in the upper membrane were removed and cells invading into the lower membrane were quantified by fluorescence by adding DAPI reagent 45 min before taking the reading. Data are expressed as the number of migrated cells in 10 high-power fields and are the means of results from three independent experiments each performed in triplicate. bFGF was used as positive control. RPMI 1640 containing 2% FBS was used as control. Each *bar* represents the mean ± SEM (**, *p <* 0:01; ***, *p* < 0.001).

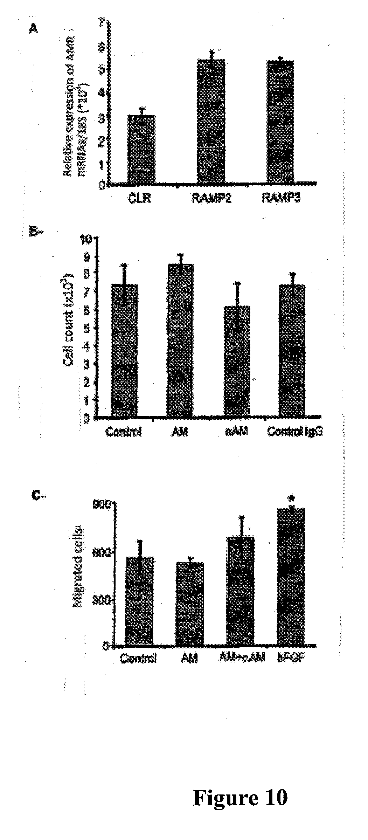

**Figure 10****: Effect of AM and αAM on LNCaP cells *in vitro.* A,** total RNA (1 µg) DNA-free from LNCaP cell line was reverse transcribed into cDNA and subjected to real-time quantitative reverse transcription (RT)-PCR for the estimation of relative CLR, RAMP2 and RAMP3 mRNAs to 18S rRNA ratio. **B**, effects of AM and αAM on LNCaP cells growth *in vitro.* For proliferation assay, LNCaP cells were seeded and treated in the presence of 5% FBS as described for PC3 cells in Fig. 9. C, no effects of AM on LNCaP cells invasion *in vitro.* For invasion assays, LNCaP cells (5 x 10⁴ cells) were seeded on a Matrigel layer in a boyden chamber assay and analyzed as described for PC3 cells in Fig. 9.

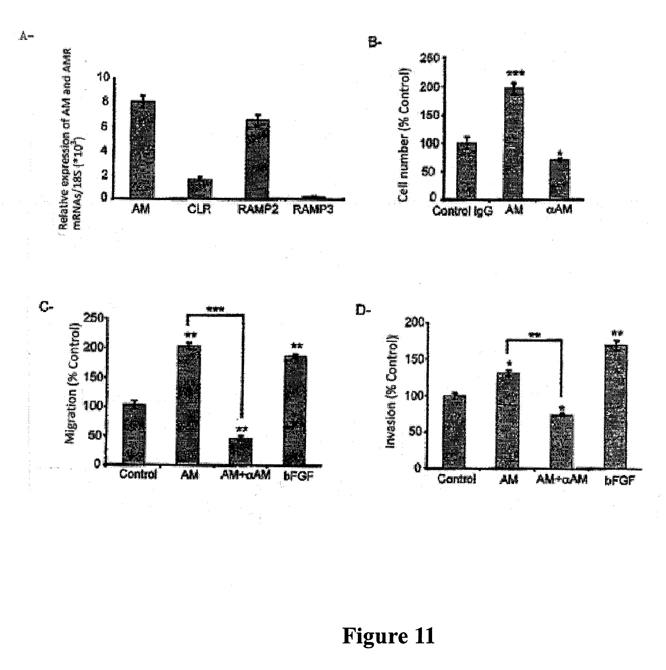

**Figure 11****: αAM blocks selective AM-mediated functions on human LEC *in vitro.*** Neonatal Human Dermal Lymphatic Microvascular Endothelial Cells (HMVEC-dLyNeo) were purchased from Lonza (Lonza, Basel, Switzerland), and expanded in microvascular endothelial cell growth medium-2 (EGM-2MV, Lonza) with growth supplements (EGM-2MV, Lonza). **A**, total RNA (1 µg) DNA-free from LECs was reverse transcribed into cDNA and subjected to real-time quantitative reverse transcription (RT)-PCR for the estimation of relative AM, CLR, RAMP2 and RAMP3 mRNAs to 18S rRNA ratio. **B**, oppose effect of AM and αAM on the growth of LECs *in vitro.* For proliferation assay, LECs were seeded at the density of 3.5 x10⁴ per well in 6 multiwell plates in the presence of EBM-2 medium supplemented with 0.5% FBS. AM (10⁻⁷ M) and αAM (70 µg/ml) were added for 6 days treatment. After treatment, cells were exposed to trypsin and were prepared for cell number counting in a coulter counter. Each bar represents the mean ± SEM of three independent experiments (* *p* <0.05; **** p* <0.001). **C** & **D**, AM stimulates migration and invasion of LECs. LECs (3x10⁴ cells) pre-treated for 30 min with αAM (70 µg/ml) were placed in the upper compartment of a 24-well Boyden chamber. AM (10⁻⁷ M) or bFGF (10⁻⁶ M) was added in lower wells. Cells migrating through the filter were counted after 20h (**C**) and those migrating in presence of Matrigel were counted after 48 h (**D**). Data are expressed as the number of migrated cells in 10-high-power fields, and the values represent the mean ± SEM of three independent experiments each performed in triplicate (* *p* < 0.05; ***p* < 0.01; ****p* < 0.001). Taken together, these data demonstrate that AM induces proliferation, migration and invasion of LECs which is impaired upon αAM treatment.

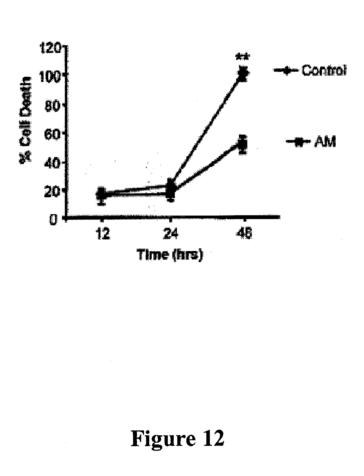

**Figure 12****. AM is a growth factor survival for lymphatic endothelial cells.** Time course of LEC death after DMEM serum deprivation in the absence and presence of AM (10⁻⁷M). Exponentially growing cells were changed into serum-free medium, and all floating cells after 12, 24 and 48h were counted with a coulter counter. Each point and bar represents mean ± SEM (n=6); values were normalized to the total number of floating dead cells 48h after serum deprivation in the absence of AM, which was set at 100%.**, *p* < 0.01.

### EXAMPLE: ADRENOMEDULLIN BLOCKADE INHIBITS TUMOR-ASSOCIATED ANGIOGENESIS AND LYMPHANGIOGENESIS IN PROSTATE CANCER HORMONE INDEPENDENT

### 1) Materials and Methods

**Cultured cells:** The human-derived CaP cell lines LNCaP androgen-dependent, PC3 and Du145 androgen-independent were obtained from American Type Culture Collection (Rockeville, MD) and cultured in RPMI 1640 (Invitrogen Life Technologies Inc., Paris, france) as previously described (4). The lymphatic endothelial cells (LECs) were obtained from Lonza (Basel, Switzerland) and cultured in EGM-2MV complemented with 5% FBS and growth factors.

**Human prostate specimens:** Human prostate samples from CaP (n=8) of high Gleason's scores were obtained from the Department of Urology (AP-HM, Marseille, France). All of the tissue procurement protocols were approved by the relevant institutional committees (Aix-Marseille Université) and were undertaken under informed consent of each patient or their relatives.

**RNA preparation and analysis:** Total RNA was prepared from Du145, PC3, LNCaP cells and LECs, reverse transcribed to cDNA and quantified as described (29).

**Development and characterization of anti-human AM polyclonal antibody:** The anti-human AM polyclonal antibody (αAM) was developed and characterized as previously described by Ouafik *et al.,* 2002 (15). Particularly, the polyclonal antibody against human AM was developed by use of the synthetic peptide corresponding to the entire AM₁₋₅₂ amide peptide (Bachem, Switzerland). Female New Zealand rabbits received injections at multiple subcutaneous sites with 300 µg of synthetic peptide emulsified with complete Freund 's adjuvant. Then the rabbits were further immunized at 2.5-week intervals with 120 µg of AM₁₋₅₂ amide emulsified with incomplete Freund's adjuvant (42). The anti-sera obtained after the fourth booster injection were screend for anti-AM activity, and then affinity purified on rProtein A Sepharose Fast Flow columns (GE Healthcare, Europe). The anti-AM polyclonal antibody (purified IgG) showed very low cross-reactivity (<7%) with AM-related peptides such as AM₂₂₋₅₂ amide, AM₂₆₋₅₂ amide, and AM₁₃₋₃₇ amide. Clacitonin, CGRP₁₋₃₇ amide, CGRP8-37 amide, and amylin showed insignificant anti-AM antibody binding (< 0.1 %) despite some homology with AM. It was also demonstrated that anti-AM antibody blocked the binding of ¹²⁵I-AM to its cell-surface receptor on DU145 cells in a dose-related manner.

**Western blotting analysis:** Cell extracts were prepared and processed for CLR, RAMP2 and RAMP3 western blot analysis as described (29). Immunoblotting of phospho-c-Raf, phospho-MEK1/2, phospho-ERK1/2, and ERK1/2 was performed using MAPK-phospho-ERK1/2 pathway samples kit (Cell Signaling Technologie, Inc.).

**Cyclic AMP assay:** Du145 cells treated with AM (10⁻⁷-10⁻⁹ M) in the presence of IBMX (10⁻⁴ M) were prepared to measure the intracellular amount of cAMP using the cAMP enzyme immunoassay Biotrak (EIA) System (GE Healthcare) according to the supplier's protocol.

**Cell migration and invasion assays:** The migration and chemoinvasion were assessed using a modified Boyden chamber assay (7). Du145 (15x10⁻³), LNCaP (50x10⁻³), and PC3 (30x10⁻³) cells were added to the upper compartment during 6h. For LECs, (30x10⁻³) cells were added during 48h for invasion and 20h for migration.

**Cell proliferation assay:** The effects of AM, αAM and control IgG on cell proliferation was examined at the indicated time points as described (19).

**Experimental protocol for animals:** Athymic NMRI *(nu*/*nu)* nude mice (Harlan, Gannat, France) were purchased at four weeks of age. Respectively 5 x 10⁶ and 1 x 10⁶ of Du145 cells were implanted subcutaneously in the right flank or orthotopically in the dorsal prostate in nude mice male (n=30). When the tumors were 700 ± 100 mm³ in size, animals were randomly divided into two groups. One group (*n*=20) received intraperitoneal (i.p.) injection of αAM (330 µg of purified IgG) and control group (*n*=10) received an irrelevant antibody (IgG of the same isotype). αAM half-life in nude mice was determined to be 3 to 4 days.

Tumor xenografts were analyzed as described (36). Particularly, athymic NMRI *(nu*/*nu)* nude mice (Harlan, Gannat, France) were purchased at four weeks of age. Suspension of Du145 cells (5 x 10⁶ in 200 µl PBS) were subcutaneous (s.c.) injected into the right flank of male mice (n=30). Tumors sizes were determined with a dial-caliper measurements, and the tumor volume was calculated as width x length x height x 0.5236 (for ellipsoid form). Mice with s.c. tumors greater than 1.5 cm in diameter were killed in accordance with University of Aix-Marseille animal rights Committee guidelines. αAM half-life in nude mice was determined to be 3 to 4 days. When the tumors were 700 ±100 mm³ in size, animals were randomly divided into two groups. One group (*n*=20) received intraperitoneal (i.p.) injection of αAM (330 µg of purified IgG) as a suspension in PBS in a volume of 0.2 ml every 3 days. A control group (*n*=10) received an irrelevant antibody (IgG of the same isotype). Tumors sizes were measured every 2 days, and mice were sacrificed at 12 weeks after injection. Tumors were stored in liquid nitrogen or embedded in Paraffin for pathologic studies and immunohistochemistry.

For orthotopic injections, the mice were anesthetized with i.p. injections of xylazine (10 mg/kg) and ketamine (50 mg/kg). An incision was made through the abdominal muscles to expose the bladder and seminal vesicles, which then were delivered through the incision to expose the dorsal prostate. Du145 cells (1x10⁶) were injected into dorsal lobe in a 50 µl volume. Treatment was initiated one month after tumor cell implantation with three weekly i.p. injections of 330 µg of αAM (n=10) or control IgG (n=10). Treatment was continued until the experiments were terminated. After the mice were killed, tumors were immersed in 4% paraformaldehyde, washed in phosphate-buffered saline (PBS), and processed for whole mount-staining.

For the experiments that concern LNCaP cells, mice (n=20) bearing heterotopic LNCaP tumors (∼500 mm³) were divided into two groups. A group of animals (n=10) was castrated and separated into two groups which received an i.p. injection of 330 µg of αAM (n=5) or control IgG (n=5) every three days. The same paradigm was applied to non castrated animals.

**Immunohistochemistry and image analysis:** Sections of paraffin-embedded samples of human CaP specimens and subcutaneous and orthotopic tumors were analyzed as described (4, 36). To assess the lymphatic vessels, a goat polyclonal anti-LYVE-1 antibody (1/100; R&D systems) was used. Particularly, sections of paraffin-embedded samples (4 µm) of human CaP specimens were tested for the presence of AM, CLR, RAMP2, RAMP3 and NSE after heat-induced antigen retrieval in citrate buffer (pH 6) at 97°C for 40 min. Optimal dilution for anti-AM (4) and anti-CLR (36) antibodies was 1/2000, anti-RAMP2 (36) antibody was 1/750, anti-RAMP3 (36) antibody was 1/1500 and anti-NSE antibody (Dako, Glostrup, Denmark) was 1/200. Incubation was maintained overnight at 4°C and followed by a second layer containing biotynylated anti-rabbit antiserum (Histostatin plus, Zymed). The next treatment was with avidin-biotin peroxidase complex kit (Histostatin plus, Zymed). Note that endogen biotin and endogen peroxidases were respectively neutralized by a biotin-blocking system (Dako, Glostrup, Denmark) and 3% H₂O₂.

Paraffin blocks of subcutaneous and orthotopic tumors were cut to 6µm sections and stained with hematoxylin and eosine (H&E) for morphology evaluation. Immunohistochemistry was carried out using the Vectastain Elite ABC Kit (Vector Laboratories, Burlingame, CA, USA). Thin (6µm) sections were incubated with anti-von Willebrand factor antibody (anti-vWF) (1:400; Dako, Glostrup, Denmark), or anti-α-Smooth muscle actin antibody (anti-αSMA) (1/80; Dako, Glostrup, Denmark) were applied to assess tumor vascularity, or a goat polyclonal anti-LYVE-1 antibody (1/100; R&D systems) to assess the lymphatic vessels. The primary antibodies were detected with the appropriate Alexa Fluor 488 or 568 secondary antibody conjugates (Molecular Probes/Invitrogen Life Technologies). All fluorescently labeled samples were mounted with Vectashield mounting medium containing 4',6-diamidino-2-phenylindole (DAPI) (Invitrogen Life Technologies) and analyzed with a compound fluorescent microscope. For nonimmunofluorescence staining, detection was carried out using a DAB chromogen. Negative control slides were obtained by omitting the primary antibody.

**Statistical analysis:** Data are expressed as mean ± SEM from at least three independent experiments. One-way analysis of variance (ANOVA) or Fisher's PLSD test (Statview 512; Brain Power Inc., Calabasas, CA, USA) was used for statistical analysis. A *p* value < 0.05 was considered significant and is indicated with an asterisk in the figures. A double and triple asterisk indicates *p* < 0.01 and *p* < 0.001, respectively.

### 2) Results

### Immunocytochemistry of AM, CLR, RAMP2, RAMP3 and NSE proteins:

The expression and localization of the NSE, AM, CLR, RAMP2 and RAMP3 proteins were evaluated by immunohistochemistry (Fig. 1). Serial sections of human prostate cancers specimens were labelled with antibodies generated against AM, CLR, RAMP2, RAMP3 and NSE. Overt NSE labelling stromal cells with weak epithelial labelling can be observed (Fig. 1*B*). Cancer specimens displayed heavily AM labelling specifically detected in the cytoplasm of epithelial cells as we reported previously (4), whereas no staining was observed in stromal cells. Overt CLR, RAMP2 and RAMP3 labelling of carcinomatous epithelium was observed (Fig. 1*D, E* and *F*)*.* Dispersed among the stromal collagen septa, numerous clusters of labelled cells for CLR, RAMP2 and RAMP3 were consistently found (Fig. 1*D, E* and *F*)*.* Sections proceeding from CaP and previously neutralized with the corresponding peptide displayed both the epithelial and the stromal compartments completely unlabeled (Fig. 1*G* and *H*)*.*

### Exogenous AM stimulates androgen-independent cancer cell line growth, cAMP activity and invasion:

The androgen independent Du145 cell line expressed AM₁ and AM₂ receptors (Fig. 8). It was examined whether AM increases intracellular cAMP, the major second messenger of AM (10, 28) in cultured Du145 cells. AM dose-dependently increased the cAMP levels in Du145 cells (Fig. 2A). AM (10⁻⁷ M) induced 2.8 ± 0.14 fold (*p* ≤ 0.001) increase in cAMP content and reached a maximum by 5 min (Fig. 2A). Incubation of Du145 cells with graded concentration of AM (10⁻⁹-10⁻⁷ M; 5 min) induced a dose-dependent stimulation of cAMP formation (Fig. 2*A*), a signal transduction pathway known to modulate cellular growth, suggesting that AM might be involved in Du145 cells growth. Accordingly, AM at 10⁻⁷ M significantly stimulated the proliferation of Du145 cells by 51 ± 9.9% (*p* < 0.01) and 96 ± 14.6% (*p* ≤ 0.001) by 6 and 8 days of treatment, respectively, when compared with untreated cells (Fig. 2*B*). To examine the possibility that AM influences prostate cancer cells in an autocrine manner, we examined the effects of αAM on basal prostate androgen-independent cancer cell functions. αAM (70µg/ml) significantly (*p* < 0.01) reduced basal cell proliferation by 33.8 ± 3.0% and 39.8 ± 12% by 6 and 8 days of treatment, respectively, when compared to untreated cells (Fig. 2*B*). In contrast, 70 µg/ml of the IgG control of irrelevant specificity showed no inhibition of cell growth (Fig. 2*B*). Taken together, these data strongly suggest that AM acts as an autocrine regulator of androgen-independent Du145 cells. PC-3 cells showed no proliferative responses either after treatment with extrinsic AM or αAM suggesting that AM system is not involved in the growth of PC-3 cells *in vitro* (Fig. 9).

Next, it was analyzed the effect of exogenous addition of AM on Du145 cell invasion. The addition of AM (10⁻⁷ M) to the bottom wells increased the number of invading cells. The data demonstrated that AM induced significant effects on invasion reaching 92 ± 22% (*p* < 0.001) compared with control (Fig. 2*C*). The effect of AM on invasion was nearly completely inhibited when cells were preincubated with a neutralizing αAM (Fig. 2*C*). The effect of AM on invasion was similar to the one observed with bFGF (10⁻⁶ M) (Fig. 2*C*). These data indicate that prostate androgen independent Du145 cancer cells are sensitive and responsive to AM stimulus. Interestingly, the same finding was obtained with PC-3 cells for which AM induced invasion by 144 ± 7.6% (*p* < 0.001). Pre-incubation with αAM decreased the induction to 74 ± 3.8% (*p* < 0.01) suggesting that the endogenous AM might be involved in the invasion process of PC-3 cells presumably by autocrine/paracrine manner (Fig. 9). Treatment of LNCaP cells with maximally active concentrations of AM at 2x10⁻⁷ M resulted in a slight decrease in the growth rates than did the control cells (Fig. 10). No effect of AM nor αAM can be observed on LNCaP cells invasion (Fig. 10).

### Adrenomedullin mediates phosphorylation of MAPK:

ERK and Akt is known to regulate cell proliferation and this signaling pathway was reported to function downstream of the AM/cAMP pathway (37, 38). Therefore, it was investigated the activity (phosphorylation) of ERK and Akt pathways in cultured Du145 cells after stimulation with AM. It was first measured the phosphorylation of cRaf and it was found that AM (5x10⁻⁷ M) increased cRaf phosphorylation as early as 2 min and declines to reach the control levels up to 10 min (Fig. 2*D*). To investigate MAPK activity, it was measured the phosphorylation of MEK1/2 and the phosphorylation of two MAPK: ERK1 (p44^{MAPK}) and ERK2 (p42^{MAPK}). The levels of p-MEK and p-ERK in Du145 cells was significantly increased as early as 2 and 5 min, respectively after the start of AM (5x10⁻⁷ M) stimulation (Fig. 2*D*). These effects sustained to be higher than control levels for up 30 min. In contrast; the level of p-Akt in Du145 cells was not markedly increased by AM (not shown). In addition, the p-ERK induced by AM was completely inhibited by MEK inhibitor, U0126 (10 µM, 30 min) (Fig. 2*D*). These results suggest that AM-induced cell proliferation is mediated at least in part by the cAMP/MEK/ERK pathway.

### αAM inhibits growth of androgen-independent prostate cancer tumor xenografts:

It was further wished to analyze the effect of endogenous AM on tumors developed *in vivo* in immunodeficient mice. We sought to determine whether AM is just a classic growth factor involved in tumor cell proliferation *in vivo,* or it has more complex role to sustain tumor growth by performing a stable angiogenesis and lymphangiogenesis to produce a functional blood and lymphatic vessels. In the first series of experiments, tumor cells were injected subcutaneous (s.c.) in the flanks of male athymic mice *(nu*/*nu),* and once tumors reached a size of 700 ± 100 mm³, mouse started to receive i.p. injection of 330 µg of αAM or control rabbit IgG of irrelevant specificity every 3 days for 4.85 consecutive weeks. Tumor growth was suppressed in αAM-treated tumors (*p* < 0.001) (Fig. 3A). At day 22, tumors in the αAM-treated mice reached a mean size of ∼421 ± 179 mm³, whereas the tumors in the control group exhibited a mean size of 2,612 ± 300 mm³.

The effect of αAM on tumor growth was tested by using the Du145 orthotopic model. As compared with the s.c. model for tumor growth, the orthotopic model requires implantation of tumor cells directly in the mouse prostate. Mice received i.p. injection of 330 µg of αAM or control IgG three times a week until sacrifice for analysis (Fig. 3*B*). In all experiments, antibody treatment showed a prominent effect on the health of the mice. Ten weeks after orthotopic tumor-cell implantation, mice in the control group appeared sickly and cachectic, characterized by sluggishness, an unkempt appearance. At the same time point, antibody-treated mice appeared healthy and active and maintained normal grooming behavior. After 6 weeks treatment, animals were sacrificed and tumor burden was assessed. Nine of ten mice treated with control IgG developed fulminant disease in which the majority of prostate tissue was replaced with tumor tissue (Fig. 3*B* and *C*). In contrast, 6 of 10 αAM-treated animals showed a dramatically response, exhibiting only small tumors (2 of 6 animals) or a complete absence of tumors (4 of 6 animals) upon gross inspection (*p* < 0.001; Fig. 3*B*). Tumor weights were significantly lower in αAM-treated animals compared to control IgG-treated animals (*p* < 0.001), further documenting tumor suppression (Fig. 3*B*).

### In the absence of androgen, αAM inhibits LNCaP tumor growth in vivo:

Following castration, LNCaP xenografts grew with androgen-independent manner and present a growth pattern comparable to one observed for hormone-independent cells such as Du145 cells. To further investigate whether the endogenous AM expressed upon castration might be involved somehow in tumor growth in castrated animals, i.p. administration of αAM or control IgG was given to castrated and non-castrated animals bearing heterotopic LNCaP tumor xenografts a three times a week. The growth of xenografts was significantly decreased by αAM treatment in castrated animals as compared to control IgG group (Fig. 4*A*). αAM treatment of non castrated animals showed no inhibition of tumor growth as compared to IgG-control group suggesting strongly that in the presence of androgen tumor growth is AM-independent (Fig. 4*B*).

### AM blockade depletes endothelial cells and pericytes in tumors:

Histological examination of Du145 heterotopic (Fig. 5*A*) and orthotopic (Fig. 5*B*) αAM-treated tumors showed a markedly decreased vessel density when compared to control IgG-treated tumors. Immunostaining of tumors with antibodies for vWF demonstrated that αAM-treated tumors were significantly less vascular than control tumors. Co-staining with anti-vWF and anti-α-SMA antibodies demonstrated that both cell types are sparce, and the vascularization is deeply disrupted and characterized by an overall reduction of endothelial cells and pericytes. Quantification of vWF stained endothelial cells and α-SMA stained pericytes demonstrate a clear decrease of both cell types in αAM-treated tumors when compared to control IgG-treated tumors (*p* < 0.001; Fig. 5*A* and *B).* In contrast, control IgG-treated tumors showed a well organized vascularization. It's of interest to note that the vascularization in normal tissues was not disrupted by αAM treatment (not shown). Immunostaining with CD31 and αSMA markers demonstrates a disruption of blood vessels in LNCaP xenografts in αAM-treated castrated animals as compared to control group (not shown).

### αAM blocks the development of tumor-associated lymphangiogenesis:

Lymphatic vessel growth is associated with a number of pathological conditions, including tumor metastasis and inflammation (39-41). To determine whether αAM treatment inhibits tumor-associated lymphangiogenesis, primary tumors from Du145 orthotopic tumor-bearing animals treated with αAM or control IgG were evaluated for tumor-associated lymphatic vessels by immunostaining for LYVE-1 (lymphatic vessel endothelial receptor 1) (Fig. 6). The surface area of tumoral lymphatic vessels was markedly reduced in tumors from αAM-treated animals compared with control IgG-treated animals. Importantly, non-tumor-associated LYVE-1 positive lymphatic vessels detected in the normal mice tissue adjacent to the tumor periphery remained unaffected by the αAM treatment. Interestingly, *in vitro* studies on human LECs demonstrate that αAM blocks selective AM-mediated functions such as proliferation, migration and invasion (Fig. 11).

Also, the smooth muscle cells, surrounding the collecting vessels in tumor tissue were eradicated in αAM-treated tumors compared to control-IgG treated tumors. Furthermore, disintegration between lymphatic endothelial cells occurred in the lymphatic vessels of αAM-treated tumors but not in the lymphatic vessels of control-IgG-treated tumors. Quantitative evaluation of the number of lymphatic vessels revealed a significant reduction specifically in αAM-treated tumor tissues, compared with in control IgG-treated tumors (*p* < 0.001; Fig. 6*A*). Interestingly, no significant difference can be observed for the number of lymphatic vessels in peritumoral tissues between IgG-control and αAM-treated animals suggesting that αAM treatment does not impede pre-existing or non-tumor associated lymphatic vessels (Fig. 6*A*). Quantitative evaluation of the number of αSMA mural cells by antibody staining revealed an overall reduction of mural cells of 46 ± 1.9% specifically in tumor tissue, compared with mural cells in control IgG-treated tumors (*p <* 0.01; Fig. 7*B*)*.*

To address the question of the reduction of lymphatic vessels number upon αAM treatment, it was hypothesized that AM is necessary somehow for lymphatic endothelial cells (LECs) survival suggesting that αAM treatment may induce LECs death by apoptosis. The apoptotic cells, as revealed by immunostaining with Mab 7-26 antibody (ssDNA), were predominantly located within the vascular lining and costaining with an antibody for LYVE-1 identified them as LECs. The apoptotic index of the αAM-treated tumors increased with treatment and reached a significant increase of ∼6-fold when compared with control tumors (*p* < 0.001; Fig. 7). Furthermore, when medium was deprived of serum, LECs cultures always contained a fraction of floating cells, almost all floating cells showed morphological features characteristic of apoptosis, such as membrane blebbing and cellular shrinkage. A significant number of cells (approximately 16%) started to float 12h after serum-deprivation, which increased markedly (84%) after 48h. The addition of AM (10⁻⁷ M) prevented the apoptosis after serum deprivation, which persisted during 48h (Fig. 12). Many adherent cultured cells also showed nuclear and cellular fragments when deprived of serum. When the cells were stained with antibody against single-stranded DNA and hematoxylin, immunohistochemically positive cells coincided very well with those showing cellular and nuclear fragments (Fig. 12). Addition of AM (10⁻⁷ M) markedly suppressed apoptotic cell death of adherent culture (Fig. 12). Taken together, these findings indicate that αAM treatment can (i) destroy peritumoral lymphatic vessels and (ii) prevent the tumor from inducing lymphangiogenesis.

### 3) Discussion

Progression of CaP towards androgen-independent status is an oncological challenge. The mechanisms responsible for the transformation of this cancer are not well understood. It was previously observed that AM was expressed in CaP (4). In the current study, it was further investigated the potential role of AM as an autocrine/paracrine factor in the disease. It was demonstrated the presence of AM and AM receptors (AM₁ and AM₂ receptors) suggesting the possibility of AM being an autocrine/paracrine growth factor in androgen-independent cancer of prostate.

To examine the possibility of an autocrine effect of AM on androgen-independent CaP Du145 cells, it was determined the effect of exogenous addition and inhibition of AM *in vitro.* The above data showed that AM significantly increased Du145 cell proliferation, invasiveness, stimulation of cAMP, and the activation of the Erk-mitogen-activated protein kinase (MAPK) pathway. These data indicate that hormone independent CaP cells are able to respond to AM in ways that would expected to further the aggressiveness of androgen-independent CaP. Importantly, it was further found that αAM inhibited the basal levels of CaP androgen-independent cell proliferation and invasion *in vitro.* These data indicate that inhibition of the interaction of AM produced by Du145 cells with cellular receptors reduces these cellular functions. Taken together, these data strongly support the conclusion that AM can act in an autocrine manner in androgen-independent CaP. The presence of autocrine loop that involves AM and its own receptors suggests that foci of AM-producing cells in a tumor could stimulate cells expressing AM receptors via autocrine/paracrine mechanisms.

The presence of AM receptors in CaPs confirms that AM must play a role *in situ.* The important role of AM in tumor progression by promoting tumor proliferation, inhibiting apoptosis (22) and promoting angiogenesis (7, 8, 15, 16) has been well established. All these activities may be relevant in prostate cancer. To examine tumor growth in response to αAM therapy, it was developed heterotopic xenograft of Du145 cells. The above results demonstrated that the αAM could be efficiently delivered *in vivo* and significantly suppress the growth of established Du145 xenografts. After 15 days of treatment, tumors in mice treated with control IgG grew progressively to a size that led to sacrifice, whereas the volume of the αAM-treated tumors was stabilized and reached at least 80% decrease when compared with the controls. AM has been shown to be involved in the multistep process of angiogenesis (7, 22). After αAM treatment for twenty days, the immunostaining of tumor sections with anti-vWF antibody demonstrated that more than 84% of the vessels disappeared with a clear decrease of the number of endothelial cells, the data demonstrate that the density of vessels with lumen was decreased in the antibody-treated tumors suggesting strongly that AM system must be involved in neovascularization and/or vessel stabilization as it was previously demonstrated for xenografts of glioblastoma (U87), lung (A549) and colorectal cancer (HT-29) (15, 36). Since Du145 cell growth *in vitro* is inhibited by αAM, the inhibition growth of the corresponding tumor xenografts *in vivo* could be a result of a combined effect on tumor cell growth associated with the effect on tumoral neoangiogenesis. The inhibition of tumor growth by αAM treatment of LNCaP xenografts in castrated animals reinforce the data obtained with Du145 xenografts and brought strong evidence demonstrating that endogenously expressed AM might be one of key players in tumor growth following androgen ablation. These data highlight the key role that may have AM in the absence of androgen by its effect on tumor cell growth as well as on tumor microenvironment by inducing and stabilizing a neovascularization leading the nutrient and oxygen supply. Interestingly, the normal vascularization in normal tissues was not affected by αAM treatment suggesting that AM system (ligand and its receptors) is well expressed and activated as well in pathological situation such as tumor growth or physiological events such as wound healing or menstrual cycle.

It is well known that subcutaneous tumors have a different vascularity than orthotopic tumors, in the purpose to evaluate the effect of αAM in orthotopic model, it was developed orthotopic xenograft of human prostate cancer of androgen-independent Du145 cells. Treatment with αAM caused a clear inhibition of tumor growth characterized at the histological level by a vascular disruption with decreased microvessel density as observed in heterotopic xenografts. However, it was observed that subcutaneous. tumors are more vascularized than orthotopic tumors even in control tumors. To determine whether lymphatic vessels might be dominant in CaP orthotopic xenografts rather than blood vessels, it was analyzed the lymphatic vessels using a murine LYVE-1 antibody. LYVE-1⁺ lymphatic vessels were observed within the tumor in control-treated specimens and were completely devoid within the αAM-treated tumors. Importantly, αAM treatment was not observed to impair pre-existing lymphatic vessels as the density of LYVE-1-positive lymphatic endothelium detected in the normal tissue was identical in control versus treated animals. Prolonged inhibition does not affect adult lymphatic vessels, indicating that AM/AM₁ and AM/AM₂ signalling is not required for the maintenance of the lymphatic vasculature in adulthood. This suggests that αAM specifically targets tumor lymphatic vessels but not normal lymphatic vessels underlying the important role of AM towards LECs in terms of proliferation, migration, invasion and organization and/or stabilization of functional lymphatic vessels during tumor growth.

### REFERENCES

**1.** Bostwick, D. G. Urology, 34: 16-22, 1989.
**2.** Bostwick, D. G. et al. Cancer, 83: 1995-2002, 1998.
**3.** Feldman, B. J. and Feldman, D. Nat Rev Cancer, 1: 34-45,2001.
**4.** Rocchi, P., et al. Cancer Res, 61: 1196-1206, 2001.
**5.** Kitamura, K., et al. Biochem Biophys Res Commun,192: 553-560, 1993.
**6.** Gibbons, C., et al. Mol Endocrinol, 21: 783-796, 2007.
**7.** Fernandez-Sauze, S., et al. Int J Cancer, 108: 797-804, 2004.
**8.** Oehler, M. K., et al. Oncogene, 21: 2815-2821, 2002.
**9.** Zhao, Y., et al. Oncogene, 16: 409-415, 1998.
**10.** Hinson, J. P., et al. Endocr Rev, 21: 138-167, 2000.
**11.** Lopez, J. and Martinez, A. Int Rev Cytol, 221: 1-92, 2002.
**12.** Caron, K. M. and Smithies, O. Proc Natl Acad Sci U S A, 98: 615-619, 2001.
**13.** Shindo, T., et al. Circulation, 104: 1964-1971, 2001.
**14.** Miller, M. J., et al. J Biol Chem, 271: 23345-23351, 1996.
**15.** Ouafik, L., et al. Am J Pathol, 160: 1279-1292, 2002.
**16.** Ishikawa, T., et al. Oncogene, 22: 1238-1242, 2003.
**17.** Ramachandran, V., et al. Cancer Res, 67: 2666-2675, 2007.
**18.** Keleg, S., et al. Int J Cancer, 121: 21-32, 2007.
**19.** Deville, J. L., et al. Int J Cancer, 125: 2307-2315, 2009.
**20.** Kim, W., et al. FASEB J, 17: 1937-1939,2003.
**21.** Nagaya, N., et al. Am J Physiol Regul Integr Comp Physiol, 288: R1432-1437, 2005.
**22.** Nikitenko, L. L., et al. Br J Cancer, 94: 1-7, 2006.
**23.** Zudaire, E., et al. Regul peptide, 112: 175-183, 2003.
**24.** McLatchie, L. M., et al. Nature, 393: 333-339, 1998.
**25.** Poyner, D. R., et al. Pharmacol Rev, 54: 233-246, 2002.
**26.** Fritz-Six, K. L., et al. J Clin Invest, 118: 40-50, 2008.
**27.** Ichikawa-Shindo, Y., et al. J Clin Invest, 118: 29-39, 2008.
**28.** Shimekake, Y., et al. J Biol Chem, 270: 4412-4417, 1995.
**29.** Berenguer, C., et al. Oncogene, 27: 506-518, 2008.
**30.** Sakai, K., et al. Eur J Pharmacol, 359: 151-159, 1998.
**31.** Mazzocchi, G., et al. Int J Oncol, 25: 1781-1787, 2004.
**32.** Jiminez, N., et al. Mol Carcinog, 38: 14-24, 2003.
**33.** Rocchi, P., et al. Oncogene, 23: 9111-9119, 2004.
**34.** Abasolo, I., et al. Regul Pept, 133: 115-122, 2006.
**35.** Joshi, B. H., et al. Cancer Res, 68: 9311-9317, 2008.
**36.** Kaafarani, I., et al. Faseb J, 23: 3424-3435, 2009.
**37.** Yu, Y. and Sato, J. D., J Cell Physiol, 178: 235-246, 1999.
**38.** Ilan, N., et al. J Cell Sci, 111 (Pt 24): 3621-3631, 1998.
**39.** Achen, M. G., et al. Cancer Cell, 7: 121-127, 2005.
**40.** Alitalo, K., et al. Nature, 438: 946-953, 2005.
**41.** Cueni, L. N., and Detmar, M., Lymphat. Res. Biol, 6: 109-122, 2008.
**42.** Harlow, E. Lane, D. (eds). Antibodies, A Laboratory manual. Cold Spring Harbor, Cold Spring Harbor Laboratory, pp 53-135, 1988.

## Claims

1. An *ex vivo* or *in vivo* method of monitoring the tumor growth of an androgen-independent prostate cancer in a subject, comprising the step of:
a) determining the amount of adrenomedullin and/or at least one adrenomedullin receptor selected among the proteins CLR, RAMP2 and RAMP3, in a prostate cancer cell obtained from said subject, and
b) comparing the amount determined in step a) with amounts previously obtained for the subject or with a standard,
a decrease in amount of adrenomedullin and/or an adrenomedullin receptor constituting a marker of the regression of said androgen-independent prostate cancer tumor growth and an increase in amount of adrenomedullin and/or an adrenomedullin receptor constituting a marker of the progression of said androgen-independent prostate cancer tumor growth.

2. The method according to claim 1, **characterized in that** the amount of adrenomedullin and of at least one adrenomedullin receptor is determined.

3. The method according to claim 1 or 2, **characterized in that** in step a) said tumor cell is contacted with an antibody binding adrenomedullin and/or at least one antibody binding at least one adrenomedullin receptor.

4. The method according to claim 3, **characterized in that** the antigen-antibody complexes formed in step a) is revealed by a method selected from the group consisting of EIA, ELISA, RIA, immunofluorescence or immunohistochemistry.

5. The method according to claim 3 or claim 4, **characterized in that** the antibody is obtained by immunizing an animal with the chimeric polypeptide of SEQ ID NO: 9.

6. The method according to any of claims 3 to 5, **characterized in that** the antibody is linked to an enzyme, a fluorophore, a heavy metal chelate or a radioisotope.

7. The method according to claim 1 or 2, **characterized in that** in step a) the amount of adrenomedullin and/or at least one adrenomedullin receptor in a prostate cancer cell is determined by detecting and quantifying RNA sequences coding adrenomedullin and/or an adrenomedullin receptor, by using a quantitative reverse transcriptase polymerase chain reaction assay.

8. A pharmaceutical composition comprising an anti-adrenomedullin antagonist antibody and/or at least one anti-adrenomedullin receptor antagonist antibody for use as a medicament for treating an androgen-independent prostate cancer.
